# EUROPÄISCHE PATENTSCHRIFT

(11) **EP 1 778 332 B1**
(45) Veröffentlichungstag und Bekanntmachung des Hinweises auf die Patenterteilung: **23.07.2008**
(21) Anmeldenummer: 05782827.9
(22) Anmeldetag: 01.08.2005
(51) Int. Cl.: A61M 16/16, F22B 1/28, F24F 6/10

(54) **VERDAMPFER, BEATMUNGSGERÄT SOWIE VERDAMPFUNGSVERFAHREN**
EVAPORATOR, ARTIFICIAL RESPIRATION APPARATUS AND EVAPORATION PROCESS
EVAPORATEUR, APPAREIL RESPIRATOIRE ET PROCEDE D'EVAPORATION

(30) Priorität: 04.08.2004 DE 102004037823
(43) Veröffentlichungstag der Anmeldung: 02.05.2007
(73) Patentinhaber: Viasys Healthcare GmbH, 97204 Höchberg (DE)
(72) Erfinder: REINSTÄDTLER, Jürgen, 97082 Würzburg (DE)
(74) Vertreter: Hellmich, Wolfgang
(86) Internationale Anmeldenummer: PCT/DE2005/001352
(87) Internationale Veröffentlichungsnummer: WO 2006/012878

(56) Entgegenhaltungen:
- WO-A-03/035157
- US-A- 1 853 422
- US-A- 4 418 269
- US-A- 4 511 790
- US-A- 2002 078 946

## Beschreibung

Die Erfindung bezieht sich auf einen Verdampfer der im Oberbegriff des Patentanspruchs 1 genannten Art. Ferner bezieht sich die Erfindung auf Beatmungsgeräte mit solchen Verdampfern. Schließlich bezieht sich die Erfindung auf ein Verdampfungsverfahren der im Oberbegriff des Patentanspruchs 9 genannten Art.

Verdampfer gemäß dem Oberbegriff des Patentanspruchs 1 sowie Verdampfungsverfahren gemäß dem Oberbegriff des Patentanspruchs 9 sind aus vielen Veröffentlichungen bekannt.

Beatmungsgeräte werden unter anderem in der Intensivmedizin zur maschinellen, künstlichen Beatmung bei allen Formen des Sauerstoffmangelzustandes verwendet. Um ein Austrocknen der Schleimhäute zu verhindern, werden zusätzlich Luftbefeuchter eingesetzt, die in ein Beatmungsgerät integriert sein können. Insbesondere in der Intensivmedizin werden hohe Anforderungen an die Keimfreiheit auch der Luftbefeuchter gestellt.

Ein Verdampfer für in der Intensivmedizin eingesetzte Beatmungsgeräte ist aus der DE 198 08 590 A1 bekannt. Dieser so genannte Beatmungsanfeuchter weist eine Schlauchpumpe als Dosiereinrichtung und einen elektrisch beheizten Verdampfer auf. Die Schlauchpumpe fördert Wasser aus einem handelsüblichen Wasserbeutel in der erforderlichen Menge, damit eine vorgegebene relative Atemgasfeuchtigkeit bei einer vorgegebenen Atemgastemperatur erreicht wird. Der Verdampfer stellt Wasser mit einer Temperatur oberhalb von 134°C bereit, das bei Mischung mit dem anzufeuchtenden Atemgas das Atemgas auf die vorgegebene Atemgastemperatur erwärmt. Vorzugsweise ist zwischen der Ausgangsseite des Verdampfers und einem Atemgaskanal eine thermische Isolierung vorgesehen, um eine Erwärmung des Atemgaskanals durch den Beatmungsanfeuchter auch ohne Zufuhr und Verdampfung von Wasser möglichst zu vermeiden. Die Austrittsöffnung des Verdampfers ragt vorzugsweise in den Atemgaskanal hinein. Die hohe Heizungstemperatur resultiert aus dem Wunsch, im Wasser möglicherweise vorhandene Keime abzutöten. Die Hygienevorschriften für Dampfsterilisation besagen nämlich, dass eine ausreichende Keimreduzierung erreicht wird, wenn die Keime einer Temperatur von 134°C für drei Minuten ausgesetzt sind.

Zu den Beatmungsgeräten im weiteren Sinne gehören auch so genannte CPAP-Geräte, die zur Behandlung von Apnoen (Atemstillstände) während des Schlafs dienen. Hierzu wurde die CPAP (continuous positive airway pressure)-Therapie entwickelt, die in Chest. Volume No. 110, Seiten 1077-1088, Oktober 1996 und Sleep, Volume No. 19, Seiten 184-188 beschrieben wird. Ein CPAP-Gerät erzeugt mittels eines Kompressors oder einer Turbine einen positiven Überdruck bis zu etwa 30 mbar und appliziert diesen vorzugsweise über einen Luftbefeuchter, über einen Schlauch und eine Nasenmaske in die Atemwege des Patienten. Dieser Überdruck soll gewährleisten, dass die oberen Atemwege während der gesamten Nacht vollständig geöffnet bleiben und somit keine Apnoen auftreten (DE 198 49 571 A1). Ein in Verbindung mit dem CPAP-Gerät eingesetzter Luftbefeuchter verhindert das Austrocknen der Schleimhäute des Patienten.

In der DE 199 36 499 A1 ist eine Vorrichtung zur Atemgasbefeuchtung für CPAP-Geräte beschrieben. Die Befeuchtungsvorrichtung umfasst eine aus einem Wannenelement und einem damit gekoppelten Topfteil gebildete Nachfülleinheit, die aus einem Aufstellgehäuse entnommen werden kann. Das Wannenelement und das Topfteil sind miteinander dicht verbunden. In dem Topfteil ist in Verbindung mit einer Trennwand ein Flüssigkeitsvorratsraum gebildet, welcher den überwiegenden Teil des zur Befeuchtung des Atemgases vorgesehenen Wasservorrats enthält. In dem unterhalb des Topfteils angeordneten Wannenelement ist ein separater Befeuchtungsbereich gebildet, in dem lediglich ein kleiner Teil des Wasservorrats enthalten ist. Die Höhe des Wassers im Wannenelement wird über eine Dosiereinrichtung auf einem vorbestimmten Niveau gehalten. Im Zuge der allmählichen Verdunstung des in dem Wannenelement befindlichen Wassers wird sukzessive oder kontinuierlich aus dem Flüssigkeitsvorratsraum Wasser nachgefüllt. Das Atemgas wird über eine Atemgaszutrittsöffnung durch den oberen Bereich des Wannenelements zu einer Atemgasaustrittsöffnung geblasen. Der Bodenbereich des Wannenelements wird durch eine Heizeinrichtung beheizt. Zur Steigerung der Wärmeübertragung ist der Bodenbereich des Wannenelements aus einem Werkstoff mit hoher Wärmeleitfähigkeit, beispielsweise Metall, hergestellt.

Die DE 200 10 553 U1 beschreibt einen ähnlichen Luftbefeuchter für Beatmungsgeräte, wie er in der DE 199 36 499 A1 beschrieben ist. Auch beim Luftbefeuchter aus der DE 200 10 553 U1 wird Luft über die Oberfläche eines beheizbaren Wasserreservoirs geführt. Anstelle der Nachfülleinheit, die aus einem Wannenelement und einem Topfteil besteht, wird ein Wasserbehälter verwendet, der im Wesentlichen aus einem Teil besteht. Der Wasserbehälter weist eine Befüllöffnung auf, die während des Betriebs mit einer Kappe verschlossen ist.

Der aus der DE 101 63 800 A1 bekannte Luftbefeuchter umfasst einen Vorratsbehälter sowie ein Regulierreservoir. Ein Regulierventil arbeitet als Schwimmer und verschließt bei hinreichend hohem Flüssigkeitsstand im Regulierreservoir die Öffnung zwischen Regulierreservoir und Vorratsbehälter. Das Regulierreservoir ist mit einem Heizkanal verbunden, der eine Heizzone zum Verdampfen von Wasser aufweist.

Die in der DE 101 51 397 C1 beschriebenen Verdampfer weisen einen Vorratsbehälter auf, der in einem Gehäuse befestigt ist. Die Öffnung des Vorratsbehälters zeigt im Befeuchtungsbetrieb nach unten. Neben dem Vorratsbehälter befindet sich ein Befeuchtungsraum mit einem Gasein- und einem Gasauslass. Durch einen Durchlass in einem Steg, den der untere Rand des Vorratsbehälters berührt oder durch eine Kerbe im unteren Rand des Vorratsbehälters selbst tritt Flüssigkeit aus dem Vorratsbehälter in den Befeuchtungsraum und bedeckt dessen Boden mit einem Flüssigkeitsbelag bestimmter Dicke. Dies wird auch als "Vogeltränken-Prinzip" bezeichnet. Unterhalb des Flüssigkeitsbelags ist ein Heizelement angeordnet.

Als nächstliegender Stand der Technik wird das Dokument US2002/0078946 A1 angesehen, welches einen Aerosolgenerator mit einem Flüssigkeitskanal und mehreren Heizelementen beschreibt, wobei die Heizelemente entlang des Flüssigkeitskanals im Direktkontakt mit der Flüssigkeit angeordnet sind, um di Flüssigkeit in dem Kanal zu erhitzen und zu verdampfen. Die Anwendung von Mehreren Heizelementen erlaubt, daß in dem Flüssigkeitskanal unterschiedliche Temperaturen in unterschiedlichen Bereichen des Kanals aufrechterhalten werden können. Das Dokument US2002/0078946 A1 beschreibt auch ein Verfahren zum Generieren vom Aerosol, in dem eine Flüssigkeit vernebelt wird.

Das deutsche Gebrauchsmuster 20 2004 004 115.4 beschreibt ebenfalls einen Verdampfer, der nach dem Vogeltränken-Prinzip arbeitet. Besonders vorteilhaft an diesem Verdampfer ist, dass er durch den Luftein- oder -auslass mit Wasser befüllt werden kann. Dies wird durch einen doppelwandige Vorratsbehälterbaugruppe erreicht, wobei die innere Wand als Umlenkung bezeichnet wird und den Vorratsraum mit dem Wasservorrat vom Befeuchtungsraum trennt. Der Boden des Befeuchtungsraums ist heizbar und mit einem dünnen Wasserfilm bedeckt.

Es ist Aufgabe der Erfindung einen schnellen Verdampfer, entsprechende Beatmungsgeräte sowie ein Verdampfungsverfahren anzugeben.

Diese Aufgabe wird durch die Lehre der unabhängigen Ansprüche gelöst.

Bevorzugte Ausführungsformen der Erfindung sind Gegenstand der Unteransprüche.

Die Schnelligkeit wird insbesondere dadurch erreicht, dass der Heizer in kleinere Heizelemente aufgeteilt wird.

Aufgrund der geringeren Trägheit des Verdampfers ist es vorteilhafterweise möglich, Atemluft lediglich während der Inspirations-, nicht aber während der Exspirationsphasen zu befeuchten.

Eine dünner Flüssigkeitsfilm trägt zur geringeren Trägheit des Verdampfers bei.

Bei entsprechender Wahl des Bodenmaterials verformt sich dieses reversibel, wenn ein Heizelement beheizt wird. Durch diese Verformung wird der Kalkablagerung entgegengewirkt, die Reinigung vereinfacht und es werden Hygienevorschriften (leichter) eingehalten.

Durch streifenförmige Leiter- oder Metallbahnen oberhalb und unterhalb einer Widerstandsschicht, wobei die Bahnen oberhalb der Widerstandsschicht aus einer Normalenrichtung zur Widerstandsschicht betrachtet senkrecht zu den Bahnen unterhalb der Widerstandsschicht verlaufen, kann die Widerstandsschicht auf einfache Weise in eine Vielzahl von Heizelementen untergliedert werden.

Ein dünner Flüssigkeitsfilm kann über einem Heizelement vollständig verdampft werden, wenn die Heizenergie eine gewisse Schwelle überschreitet. Oberhalb dieser Schwelle kann die Atemluft unabhängig von der Befeuchtung geheizt werden. Vorteilhaft an der vollständigen Verdunstung des Flüssigkeitsfilms über einem Heizelement ist ferner, dass die absolute Befeuchtung der Luft, oder allgemeiner, die verdampfte Flüssigkeitsmenge, unabhängig von der Geschwindigkeit des Nachlaufens der Flüssigkeit, insbesondere von der Viskosität der Flüssigkeit, wird.

Je höher die Heizleistung ist, desto eher kommt es zu einer explosionsartigen Verdampfung des Flüssigkeitsfilms. Die verdampfte Flüssigkeit "schießt" förmlich in den Gasstrom und sorgt so für eine bessere Durchmischung von Gas- und Flüssigkeitsmolekülen oder -atomen. Mit anderen Worten wird so eine gleichmäßigere Verteilung der Flüssigkeitsmoleküle oder -Atome erreicht.

Wird die Temperatur des Beatmungsschlauches bei der Befeuchtung berücksichtigt, kann ein Kondensieren von Wasser im Beatmungsschlauch verhindert werden. Das Einatmen von Wasser ist für den Patienten unangenehm. Außerdem können feuchte Stellen im Beatmungsschlauch als Brutstätten für Keime dienen. Hierzu sei noch bemerkt, dass die Temperatur in Beatmungsgeräten für die Intensivmedizin höher als die Umgebungstemperatur liegt (vgl. DE 198 08 590 A1). Deshalb sinkt die Temperatur im Beatmungsschlauch vom Beatmungsgerät zum Patienten ab.

Ähnlich wirkt ein Abschalten der Befeuchtung während der Exspiration. Zusätzlich wird hierbei Energie gespart.

Im Folgenden werden bevorzugte Ausführungsformen der Erfindung unter Bezugnahme auf die beiliegenden Zeichnungen näher erläutert. Dabei zeigen:
Fig. 1 einen vertikalen Schnitt durch einen erfindungsgemäßen Verdampfer;
Fig. 2 einen horizontalen Schnitt durch den erfindungsgemäßen Verdampfer;
Fig. 3 eine Untersicht des Bodens des erfindungsgemäßen Verdampfers; und
Fig. 4 ein Beatmungsgerät mit einem erfindungsgemäßen Verdampfer.

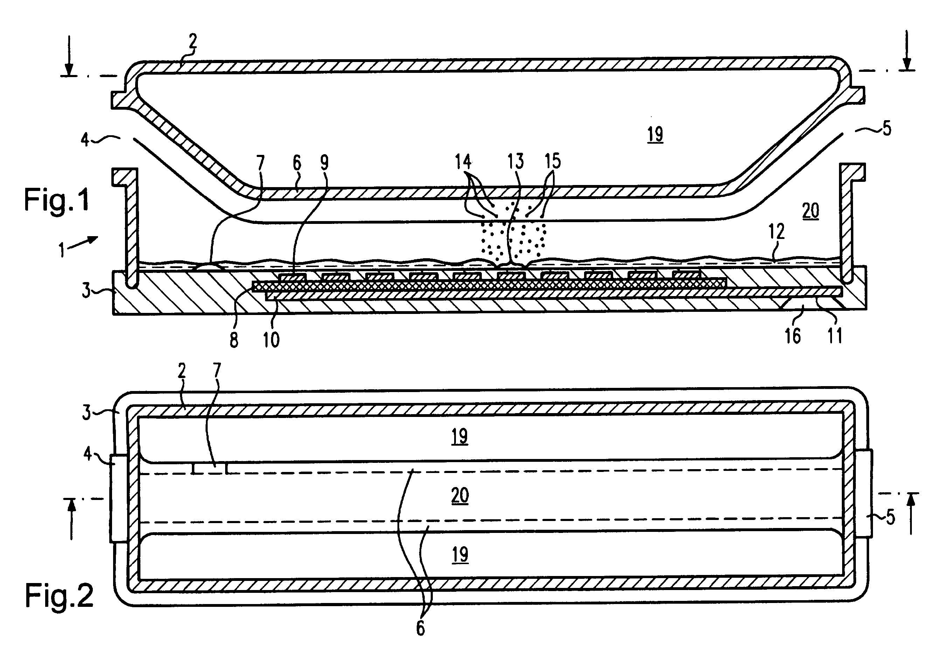

Fig. 1 zeigt einen seitlichen Schnitt durch einen erfindungsgemäßen Verdampfer 1. Der Verdampfer umfasst eine Vorratsbehälterbaugruppe 2 sowie einen Boden 3. Vorratsbehälterbaugruppe 2 bildet einen Einlass 4, einen Auslass 5 und einen Tunnel 6. Der Tunnel 6 entspricht im deutschen Gebrauchsmuster 20 2004 004 115.4 der Umlenkung 3 und kann wie die Umlenkung geformt sein, damit der Vorratsraum 19 über Einlass 4 oder Auslass 5 mit Wasser befüllt werden kann. Dies ist jedoch für diese Erfindung von untergeordneter Bedeutung.

Der Verdampfer 1 arbeitet wie die Verdampfer einiger zitierter Schriften nach dem Vogeltränken-Prinzip. In Betriebsstellung (Fig. 1) tritt durch Aussparung 7 Wasser aus Vorratsraum 19 in Befeuchtungsraum 20, bis der Wasserfilm 12 eine vorgegebene Dicke erreicht hat und damit Aussparung 7 verschließt. Nimmt die Dicke von Wasserfilm 12 durch Verdunstung oder Verdampfung - was in dieser Anmeldung synonym verwendet wird - von Wasser ab, wird Aussparung 7 wieder freigegeben, so dass Wasser aus Vorratsraum 19 nachfließen kann. So wird die Dicke von Wasserfilm 12 unabhängig vom Führerstand den Vorratsraum 19 weitgehend konstant gehalten.

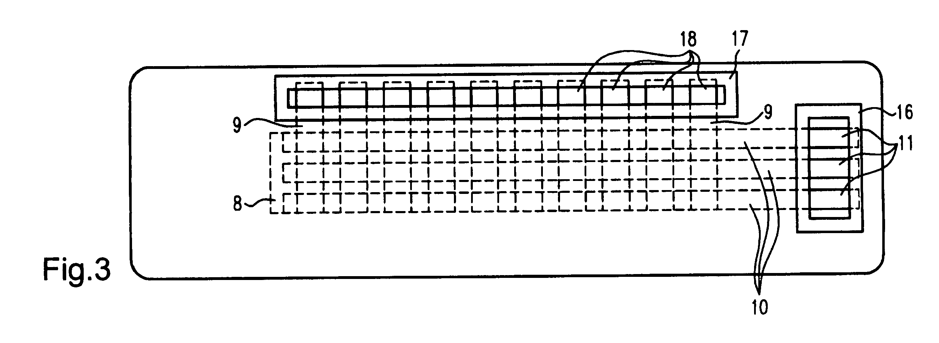

Im Boden 3 befindet sich ein Heizer, der aus einer Vielzahl von Heizelementen besteht oder in eine Vielzahl von Heizelementen untergliedert ist. In der in den Figuren dargestellten Ausführungsform besteht der Heizer aus einer Widerstandsschicht 8, oberhalb von der obere Metallstreifen 9 und unterhalb von der untere Metallstreifen 10 angeordnet sind. Von oben oder unten betrachtet, also aus einer Normalenrichtung zu der durch Widerstandsschicht 8 definierten Ebene, scheinen obere Metallstreifen 9 rechtwinklig zu unteren Metallstreifen 10 zu verlaufen (Fig. 3). Dort, wo sich ein oberer Metallstreifen 9 und ein unterer Metallstreifen 10 von unten betrachtet zu überlappen scheinen, entsteht ein Heizelement. Legt man nämlich zwischen dem oberen und unteren Metallstreifen 9 beziehungsweise 10 eine Spannung an, so fließt der resultierende Strom im Wesentlichen im Überlappungsbereich durch Widerstandsschicht 8 und erhitzt den Überlappungsbereich.

Anstelle von Metallstreifen 9 und 10 können Streifen aus einem beliebigen anderen Material verwendet werden, dessen spezifische Leitfähigkeit ausreichend hoch gegenüber der spezifische Leitfähigkeit von Widerstandsschicht 8 ist.

Oberhalb des erhitzten Heizelements 13 ist in Fig. 1 ein Nicht-Gleichgewichtszustand dargestellt. Durch starkes Erhitzen von Heizelement 13 wurde der Wasserfilm oberhalb von Heizelement 13 plötzlich verdampft. Der restliche Wasserfilm hatte noch nicht ausreichend Zeit um das verdampfte Wasser oberhalb von Heizelement 13 zu ersetzen. In Befeuchtungsraum 20 oberhalb von Heizelement 13 sind Wasserdampftropfen 14 und Kalkpartikel 15 dargestellt, die durch das explosionsartige Verdampfen des Wasserfilms entstehen. Durch das explosionsartige Verdampfen kann sich der im Wasser gelöste Kalk praktisch nicht am Boden 3 absetzen. Zusätzlich kann das Bodenmaterial, insbesondere dessen thermischer Ausdehnungskoeffizient, so gewählt werden, dass sich der Boden im Bereich eines Heizelements beim vorübergehenden Erhitzen des Heizelements reversibel verformt, so dass abgelagerter Kalk abplatzt.

Fig. 2 zeigt einen horizontalen Schnitt durch Verdampfer 1. Aussparung 7 kann sich in der Mitte von Verdampfer 1 befinden, um den Verdampfer unempfindlicher gegen Neigung zu machen.

Fig. 3 zeigt eine Untersicht von Boden 3. Gestrichelt sind die oberen und unteren Metallstreifen 9 und 10 sowie die Widerstandsschicht 8 eingetragen. Lediglich in Aussparung 16 liegen die unteren Metallstreifen 10 frei und bilden Kontaktflächen 11. In ähnlicher Weise liegen die oberen Metallstreifen 9 im Bereich von Aussparung 17 frei und bilden Kontaktflächen 18. Kontaktflächen 11 und 18 können vergoldet oder mit einem anderen edlen Metall überzogen sein um Korrosionsbeständigkeit zu gewährleisten.

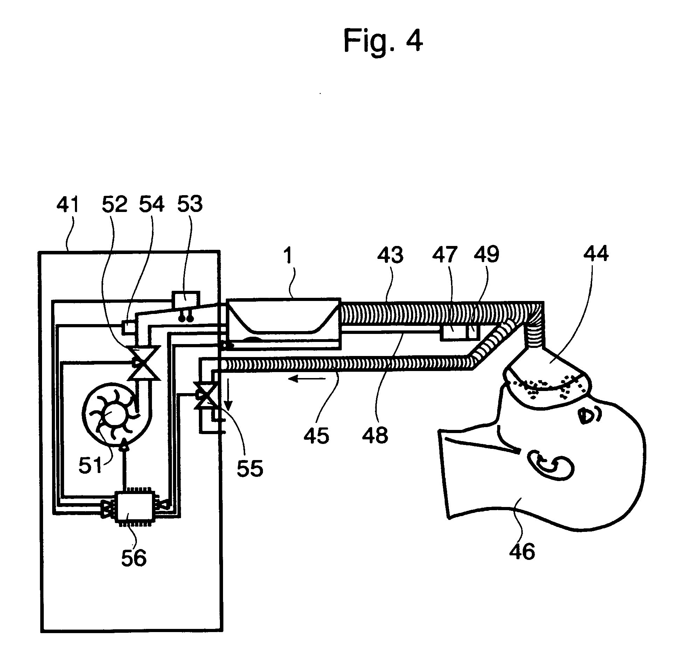

Fig. 4 zeigt ein Beatmungsgerät 41 zusammen mit einem erfindungsgemäßen Verdampfer 1, der als Luftbefeuchter arbeitet. Beatmungsgerät 41 umfasst einen Kompressor 51, der auch als Turbine, Ventilator, Verdichter oder Gebläse bezeichnet wird. Die vom Kompressor 51 geförderte Luft fließt während der Inspirationsphasen durch Ventil 52. Während der Exspiration ist Ventil 52 geschlossen, um ein Rückatmen und die damit verbundene Kontamination vom Kompressor 51 und in Förderrichtung davor liegenden Schallschutzschäumen mit vom Patienten 46 ausgeatmeten Krankheitserregern zu verhindern. Nach Ventil 52 fließt die geförderte Luft an Drucksensor 54 und Flusssensor 53 vorbei, bevor sie Verdampfer 1 zugeführt wird. Nach Verdampfer 1 wird die Luft über Beatmungsschlauch 43 und Gesichtsmaske 44 Patienten 46 zugeführt. Während der Exspirationsphasen wird Ventil 55 geöffnet, so dass die ausgeatmete Luft über Beatmungsschlauch 45 entsorgt wird.

Steuerung 56 erhält die Signale vom Drucksensor 54 und Flusssensor 53 und bestimmt hieraus Inspirations- und Exspirationsphasen. Basierend auf diesem Ergebnis werden Ventile 52 und 55, die Drehzahl von Kompressor 51 sowie Verdampfer 1, insbesondere dessen Heizelemente, gesteuert. Bei einer Ausführungsform, welche jedoch als Verfahren nicht beansprucht wird, wird Verdampfer 1 lediglich während der Inspirationsphasen beheizt, was aufgrund seiner geringen thermischen Trägheit möglich ist. Hierbei kann die Anzahl der beheizten Heizelemente in Abhängigkeit des von Flusssensor 53 gemessenen Luftflusses gewählt werden. Je höher der Luftfluss, desto mehr Heizelemente können beheizt werden. Hierbei kann man sich am mittleren Luftfluss orientieren, sodass die Anzahl der beheizten Heizelemente während einer Inspirationsphase konstant bleibt. In einer anderen Ausführungsform, welche auch als Verfahren nicht beansprucht wird, kann die Anzahl der beheizten Heizelemente zeitnäher an den gemessenen Luftfluss angepasst werden, sodass am Anfang und am Ende einer Inspirationsphase weniger Heizelemente beheizt werden, als in der Mitte der Inspirationsphase.

Auch die Frequenz des Herzens einzelner Heizelemente kann in Abhängigkeit des gemessenen Luftflusses gewählt werden. Insbesondere kann hierbei die Geschwindigkeit des nachfließenden Wassers berücksichtigt werden, sodass ein Heizelement erst dann wieder beheizt wird, nachdem die Dicke des Wasserfilms mindestens 50% Gleichgewichtsdicke erreicht hat. Zusätzlich oder alternativ kann die Heizleistung der einzelnen Heizelemente durch eine Pulsweitenmodulation gesteuert werden, deren Frequenz weit oberhalb der thermischen Zeitkonstante eines einzelnen Heizelement liegt.

Bei der Steuerung von Verdampfer 1 kann auch das Sensorsignal des Temperatursensors 47 berücksichtigt werden um ein Kondensieren im Beatmungsschlauch 43 zu verhindern.

In einer weiteren Ausführungsform kann anstelle des oder zusätzlich zu dem Temperatursensors 47 ein Feuchtesensor 49 vorgesehen sein, der die Luftfeuchte an einer Stelle in Beatmungsschlauch 43 zwischen Verdampfer 1 und Gesichtsmaske 44 misst. Sind sowohl Temperatursensors 47 als auch Feuchtesensor 49 vorhanden, kann eine gemessene relative Feuchte in absolute Feuchte und umgekehrt umgerechnet werden.

Feuchtesensor 49 und Verdampfer 1 können über Steuerung 56 zu einer Regelschleife verbunden werden, so dass die Luftfeuchte sehr genau eingestellt werden kann. In vorteilhafter Weise erfolgt diese Regelung lediglich während der Inspiration, da nur dann ein Luftfluss von Beatmungsgerät 41 zum Patienten 46 für eine geringe zeitliche Verzögerung zwischen Verdampfer 1 und Feuchtesensor 49 sorgt und dadurch eine schnelle Regelung ermöglicht. Ferner kann die Zeitkonstante der Regelungsschleife in Abhängigkeit des von Flusssensor 53 gelieferten Signals eingestellt werden: je höher der Fluss, desto schneller die Regelung.

In einer weiteren Ausführungsform kann die Gastemperatur und/oder die Luftfeuchte vor Verdampfer 1 gemessen werden und der Verdampfer sowie die verdampfte Flüssigkeitsmenge entsprechend gesteuert werden, sodass nach dem Verdampfer die gewünschte Luftfeuchte erreicht wird. Dies kann zusätzlich oder alternativ zu einer Messung durch die Sensoren 47 und 49 erfolgen.

Zwar wird ein erfindungsgemäßer Verdampfer in den meisten Fällen als Luftbefeuchter betrieben werden. Der Schutzbereich soll jedoch nicht hierauf beschränkt werden, da ja auch ein Gasgemisch mit einem von 210 mbar abweichenden Sauerstoffpartialdruck befeuchtet werden kann und/oder anstelle von Wasser beispielsweise ätherische Öle verwendet werden können.

Anstatt einem Beatmungsgerät 41 für intensivmedizinische Anwendungen kann ein erfindungsgemäßer Verdampfer aufgrund seines geringen Leistungsbedarfs und seiner geringen thermischen Trägheit auch vorteilhaft zusammen mit CPAP- oder BiLevel-Geräten eingesetzt werden.

Die Erfindung wurde zuvor anhand von bevorzugten Ausführungsformen näher erläutert. Für einen Fachmann ist jedoch offensichtlich, dass verschiedene Abwandlungen und Modifikationen gemacht werden können. Deshalb wird der Schutzbereich durch die nachfolgenden Ansprüche und ihre Äquivalente festgelegt.

### Bezugszeichenliste

- 1: Verdampfer
- 2: Vorratsbehälterbaugruppe
- 3: Boden
- 4: Einlass
- 5: Auslass
- 6: Tunnel
- 7: Aussparung
- 8: Widerstandsschicht
- 9: obere Metallstreifen
- 10: untere Metallstreifen
- 11: Kontaktflächen
- 12: Wasserfilm
- 13: erhitztes Heizelement
- 14: Wasserdampftropfen
- 15: Kalkpartikel
- 16: Aussparung
- 17: Aussparung
- 18: Kontaktflächen
- 19: Vorratsraum
- 20: Befeuchtungsraum
- 41: Beatmungsgerät
- 43: Beatmungsschlauch (Inspiration)
- 44: Gesichtsmaske
- 45: Beatmungsschlauch (Exspiration)
- 46: Patient
- 47: Temperatursensor
- 48: elektrische Leitung
- 49: Feuchtesensor
- 51: Kompressor
- 52: Ventil
- 53: Flusssensor
- 54: Drucksensor
- 55: Ausatemventil
- 56: Steuerung

## Patentansprüche

1. Verdampfer mit:
einem Gehäuse (2, 3) zur Aufnahme einer Flüssigkeit; und
einem Heizer (8, 9, 10), wobei das Gehäuse so geformt ist und der Heizer im Gehäuse in Betriebsposition so angeordnet ist, dass sich im Betrieb auf dem Heizer ein dünner Flüssigkeitsfilm (12) ausbildet; und
der Heizer eine Vielzahl von Heizelementen (13) aufweist, die mit einer Steuereinrichtung (56) elektrisch verbunden sind;
**dadurch gekennzeichnet, dass**
die Heizelementen (13) von der Steuereinrichtung (56) einzeln beheizt werden können, und
die Steuereinrichtung (56) so ausgelegt ist, dass die Steuereinrichtung (56) ein bestimmtes Heizelement (13) mit so viel Leistung versorgen kann, dass der Flüssigkeitsfilm über dem bestimmten Heizelement (13) vollständig verdampft.

2. Verdampfer nach Anspruch 1, **dadurch gekennzeichnet, dass das** Gehäuse zwei Anschlüsse, nämlich einen Einlass (4) und einen Auslass (5) aufweist, wobei der Einlass (4) dem Zuführen von Gas und der Auslass (5) zum Abführen von Gas dient, und wobei das abgeführte Gas mit Flüssigkeitsmolekülen oder -atomen angereichert ist.

3. Verdampfer gemäß Anspruch 1 oder 2, **dadurch gekennzeichnet, dass** der Heizer eine Widerstandsschicht (8) aufweist, die oben und unten durch einen oder mehrere Metallstreifen (9, 10) kontaktiert ist, wobei die oberen und unteren Metallstreifen (9, 10) aus der Normalen zur Widerstandsschicht betrachtet etwa rechtwinklig zueinander verlaufen, sodass im Überlappungsbereich eines oberen und unteren Metallstreifens ein Heizelement gebildet wird.

4. Beatmungsgerät mit:
einem Kompressor (51) zum Fördern von Gas;
einem Verdampfer (1) gemäß Anspruch 2, dessen Einlass (4) mit dem Kompressor (51) verbunden ist und dem über die Verbindung Gas zugeführt wird;
einem Beatmungsschlauch (43), dessen beatmungsgerätseitiges Ende mit dem Auslass (5) des Verdampfers verbunden ist und der ein patientenseitiges Ende aufweist; und
einem Temperatursensor (47), der in thermischem Kontakt mit dem Beatmungsschlauch (43) steht; und
der Steuereinrichtung (56), der das Signal des Temperatursensors zugeführt wird und die die Temperatur der einzelnen Heizelemente (13) so steuert, dass im Beatmungsschlauch kein Wasser kondensiert.

5. Beatmungsgerät gemäß Anspruch 4, **dadurch gekennzeichnet, dass** das Beatmungsgerät ferner einen Flusssensor (53) aufweist, der den Fluss des geförderten Gases bestimmt; wobei das Signal des Flusssensors (53) der Steuereinrichtung (56) zugeführt wird, die aus diesem Signal Inspirations- und Exspirationsphasen bestimmt und die die Heizelemente (13) nur während einer Inspirationsphasen beheizt.

6. Beatmungsgerät mit:
einem Kompressor (51) zum Fördern von Gas;
einem Verdampfer (1) gemäß Anspruch 2, dessen Einlass (4) mit dem Kompressor (51) verbunden ist und dem über die Verbindung Gas zugeführt wird;
einem Flusssensor (53), der den Fluss des geförderten Gases bestimmt;
der Steuereinrichtung (56), der das Signal des Flusssensors (53) zugeführt wird, die aus diesem Signal Inspirations- und Exspirationsphasen bestimmt und die die Heizelemente (13) nur während einer Inspirationsphase beheizt.

7. Verdampfungsverfahren mit:
Ausbilden eines Flüssigkeitsfilms (12) oberhalb eines Heizers (8, 9, 10);
Heizen eines bestimmten Heizelements (13) aus einer Vielzahl von Heizelementen des Heizers (8, 9, 10), wobei sich die Flüssigkeit (12) in thermischen Kontakt mit dem beheizten Heizelement (13) befindet; und
Erhitzen einer Flüssigkeit (12), wobei ein Teil der Moleküle oder Atome der Flüssigkeit verdampft;
**gekennzeichnet durch:**
Heizen des bestimmten Heizelements (13) aus einer Vielzahl von Heizelementen des Heizers (8, 9, 10), so dass der Flüssigkeitsfilm (12) oberhalb des bestimmten Heizelements (13) vollständig verdampft.

8. Verdampfungsverfahren gemäß Anspruch 7, **gekennzeichnet durch:**
Zuführen von Luft **durch** einen Einlass (4); und
Abführen von mit Flüssigkeitsmolekülen oder -atomen angereicherter Luft durch einen Auslass (5).

9. Verdampfungsverfahren gemäß Anspruch 7 oder 8, **gekennzeichnet durch:**
Leiten von elektrischem Strom **durch** eine Widerstandsschicht (8), wobei Spannung zwischen einem Metallstreifen (9) oberhalb der Widerstandsschicht (8) und einem Metallstreifen (10) unterhalb der Widerstandsschicht (8) angelegt wird, wobei die Metallstreifen (9, 10) oberhalb der Widerstandsschicht (8) und unterhalb der Widerstandsschicht (8) aus der Normalen zur Widerstandsschicht (8) betrachtet etwa rechtwinklig zueinander verlaufen.

10. Verdampfungsverfahren gemäß einem der Ansprüche 7 bis 9, **gekennzeichnet durch:**
Messen der Temperatur (47) an einem Schlauch (43), **durch** den das mit Flüssigkeitsmolekülen oder -atomen angereicherte Gas geleitet wird; und
Begrenzen der Heizleistung des Heizelements (13), so dass keine Flüssigkeit im Schlauch (43) kondensiert.

## Claims

1. An evaporator, comprising:
a casing (2, 3) for receiving a liquid; and
a heater (8, 9, 10),
wherein the casing is formed such and the heater in the casing is arranged in an operating position such that a thin liquid film (12) is formed on the heater during the operation, and
the heater comprises a plurality of heating elements (13) which are electrically connected to a controller (56),
**characterized in that**
the heating elements (13) can be heated individually by the controller (56), and
the controller (56) is configured in such a way that the controller (56) can supply a specific heating element (13) with such an amount of power that the liquid film above the specific heating element (13) evaporates completely.

2. The evaporator according to claim 1, **characterized in that** the casing has two connections, namely an inlet (4) and an outlet (5), wherein the inlet (4) serves to supply gas and the outlet (5) serves to discharge gas, and wherein the discharged gas is accumulated with liquid molecules or atoms.

3. The evaporator according to claim 1 or 2, **characterized in that** the heater comprises a resistive layer (8) contacted by one or more metal strips (9, 10) above and underneath thereof, wherein the upper and lower metal strips (9, 10) run approximately at right angles to each other when viewed from the normal to the resistive layer so that a heating element is formed in the overlapping region of an upper and a lower metal strip.

4. A respiration apparatus, comprising:
a compressor (51) for delivering gas;
an evaporator (1) according to claim 2, the inlet (4) of which is connected to the compressor (51) and to which gas is supplied via the connection;
a respiratory tube (43), the end of which on the respiration apparatus's side is connected to the outlet (5) of the evaporator and which comprises a subject's side end; and
a temperature sensor (47) thermally contacting the respiratory tube (43); and
the controller (56), to which the signal of the temperature sensor is supplied and which controls the temperature of the individual heating elements (13) such that there is no water condensation in the respiratory tube.

5. The respiration apparatus according to claim 4, **characterized in that** the respiration apparatus moreover comprises a flow sensor (53) determining the flow of the delivered gas, wherein the signal of the flow sensor (53) is supplied to the controller (56) which determines inspiration and expiration phases from this signal and heats the heating elements (13) only during an inspiration phase.

6. A respiration apparatus, comprising:
a compressor (51) for delivering gas;
an evaporator (1) according to claim 2, the inlet (4) of which is connected to the compressor (51) and to which gas is supplied via the connection;
a flow sensor (53) determining the flow of the delivered gas;
the controller (56), to which the signal of the flow sensor (53) is supplied, which determines inspiration and expiration phases from this signal and heats the heating elements (13) only during an inspiration phase.

7. An evaporation process, comprising:
forming a liquid film (12) above a heater (8, 9, 10);
heating a specific heating element (13) from a plurality of heating elements of the heater (8, 9, 10), wherein the liquid (12) is in thermal contact with the heated heating element (13); and
heating a liquid (12), wherein a portion of the molecules or atoms in the liquid evaporates;
**characterized by:**
heating the specific heating element (13) from a plurality of heating elements of the heater (8, 9, 10) so that the liquid film (12) above the heating element (13) evaporates completely.

8. The evaporation process according to claim 7, **characterized by:**
supplying air through an inlet (4); and
discharging air accumulated with liquid molecules or atoms through an outlet (5).

9. The evaporation process according to claim 7 or 8, **characterized by:**
conducting an electric current through a resistive layer (8), wherein a voltage is applied between a metal strip (9) above the resistive layer (8) and a metal strip (10) underneath the resistive layer (8), wherein the metal strips (9, 10) above the resistive layer (8) and underneath the resistive layer (8) run approximately at right angles to each other when viewed from the normal to the resistive layer (8).

10. The evaporation process according to one of claims 7 to 9, **characterized by:**
measuring the temperature (47) on a tube (43) through which the gas accumulated with the liquid molecules or atoms is conducted; and
limiting the heating power of the heating element (13) so that no liquid condenses in the tube (43).

## Revendications

1. Évaporateur avec :
un boîtier (2, 3) destiné à recevoir un liquide ; et
un dispositif de chauffage (8, 9, 10), le boîtier étant formé de telle façon et le dispositif de chauffage dans le boîtier étant disposé de telle façon qu'un fin film de liquide (12) se forme en fonctionnement sur le dispositif de chauffage ; et
le dispositif de chauffage présentant une pluralité d'éléments chauffants (13) reliés électriquement à un dispositif de commande (56) ;
**caractérisé en ce que**
les éléments chauffants (13) du dispositif de commande (56) peuvent être chauffés séparément, et
le dispositif de commande (56) est réalisé de telle sorte que le dispositif de commande (56) peut fournir une puissance telle à un élément chauffant (13) donné que le film de liquide est entièrement évaporé sur l'ensemble de l'élément chauffant (13) donné.

2. Évaporateur selon la revendication 1, **caractérisé en ce que** le boîtier comporte deux raccords, notamment un orifice d'admission (4) et un orifice de sortie (5), l'orifice d'admission (4) servant à amener le gaz et l'orifice de sortie (5) à évacuer le gaz et le gaz évacué étant enrichi en molécules ou en atomes de liquide.

3. Évaporateur selon la revendication 1 ou 2, **caractérisé en ce que** le dispositif de chauffage comporte une couche résistante (8) contactée en haut et en bas à l'aide par une ou plusieurs bandes de métal (9, 10), les bandes de métal (9, 10) supérieure et inférieure étant approximativement perpendiculaires l'une par rapport à autre lorsque observées perpendiculairement à la couche résistante, de façon à former un élément chauffant dans la zone de chevauchement d'une bande de métal supérieure et d'une bande de métal inférieure.

4. Appareil respiratoire avec :
un compresseur (51) pour amener du gaz ;
un évaporateur (1) selon la revendication 2, dont l'orifice d'admission (4) est relié au compresseur (51) et auquel du gaz est amené par l'intermédiaire du raccord ;
un flexible respiratoire (43) dont l'extrémité située du côté de l'appareil respiratoire est reliée à l'orifice de sortie (5) de l'évaporateur et comportant une extrémité du côté du patient ; et
un capteur de température (47) en contact thermique avec le flexible respiratoire (43) ; et
le dispositif de commande (56) auquel le signal du capteur de température est amené et qui commande la température des différents éléments chauffants (13) de façon à éviter toute condensation d'eau dans le flexible respiratoire.

5. Appareil respiratoire selon la revendication 4, **caractérisé en ce que** l'appareil respiratoire comporte en outre un capteur de débit (53) qui définit le débit du gaz amené ; le signal du capteur de flux (53) étant amené au dispositif de commande (56) qui définit à partir de ce signal les phases d'inspiration et d'expiration et chauffe les éléments chauffants (13) uniquement pendant les phases d'inspiration.

6. Appareil respiratoire avec :
un compresseur (51) pour amener du gaz ;
un évaporateur (1) selon la revendication 2 dont l'orifice d'admission (4) est relié au compresseur (51) et auquel du gaz est amené par l'intermédiaire du raccord ;
un capteur de débit (53) qui définit le débit du gaz convoyé ;
le dispositif de commande (56) auquel le signal du capteur de débit (53) est amené et qui définit à partir de ce signal les phases d'inspiration et d'expiration et ne chauffe les éléments hauffants (13) que pendant la phase d'inspiration.

7. Procédé d'évaporation avec :
formation d'un film de liquide (12) au-dessus d'un dispositif de chauffage (8, 9, 10) ; et
chauffage d'un élément chauffant (13) donné parmi une pluralité d'éléments chauffants du dispositif de chauffage (8, 9, 10), le liquide (12) étant en contact thermique avec l'élément chauffant (13) chauffé ; et
réchauffement d'un liquide (12), entraînant une évaporation partielle des molécules ou des atomes du liquide ;
**caractérisé par :**
le chauffage de l'élément chauffant (13) donné parmi une pluralité d'éléments chauffants du dispositif de chauffage (8, 9, 10), de façon à entraîner l'évaporation complète du film de liquide (12) au-dessus de l'élément chauffant (13) donné.

8. Procédé d'évaporation selon la revendication 7, **caractérisé par** :
l'amenée d'air à travers un orifice d'admission (4) ; et
l'évacuation de l'air enrichi en molécules ou en atomes de liquide à travers un orifice de sortie (5).

9. Procédé d'évaporation selon la revendication 7 ou 8, **caractérisé par** :
la conduction d'un courant électrique à travers une couche résistante (8), lorsque la tension est établie entre une bande de métal (9) située au-dessus de la couche résistante (8) et une bande de métal (10) située en dessous de la couche résistante (8), les bandes de métal (9, 10) au-dessus de la couche résistante (8) et en dessous de la couche résistante (8) s'étendant approximativement perpendiculairement l'une par rapport à l'autre lorsque observées perpendiculairement à la couche résistante (8).

10. Procédé d'évaporation selon l'une quelconque des revendications 7 à 9,
**caractérisé par :**
la mesure de la température (47) au niveau d'un flexible (43) à travers lequel l'air enrichi en molécules ou en atomes de liquide est amené ; et
la limitation de la puissance de chauffe de l'élément chauffant (13) de façon à éviter toute condensation de liquide dans le flexible (43).
